# NEW EUROPEAN PATENT SPECIFICATION

(11) **EP 2 473 058 B2**
(45) Date of publication and mention of the opposition decision: **25.12.2024**
(45) Mention of the grant of the patent: 09.08.2017
(21) Application number: 10751643.7
(22) Date of filing: 01.09.2010
(51) Int. Cl.: A23C 9/12, C12N 9/12, C12R 1/46

(54) **LACTIC BACTERIUM WITH MODIFIED GALACTOKINASE EXPRESSION FOR TEXTURIZING FOOD PRODUCTS BY OVEREXPRESSION OF EXOPOLYSACCHARIDE**
MILCHSÄUREBAKTERIUM MIT MODIFIZIERTER GALAKTOKINASEEXPRESSION ZUR TEXTURIERUNG VON LEBENSMITTELPRODUKTEN MITTELS ÜBEREXPRESSION VON EXOPOLYSACCHARID
BACTÉRIE LACTIQUE PRÉSENTANT UNE EXPRESSION GALACTOKINASE MODIFIÉE POUR LA TEXTURATION DE PRODUITS ALIMENTAIRES PAR SUREXPRESSION D'UN EXOPOLYSACCHARIDE

(30) Priority: 01.09.2009 DK 200900984; 28.01.2010 DK 201000070
(43) Date of publication of application: 11.07.2012
(73) Proprietor: Chr. Hansen A/S, 2970 Hoersholm (DK)
(72) Inventor: JANZEN, Thomas, DK-2000 Frederiksberg (DK); CHRISTIANSEN, Ditte Ellegaard, DK-2720 Vanloese (DK)
(74) Representative: Hoffmann Eitle
(86) International application number: PCT/EP2010/062808
(87) International publication number: WO 2011/026863

(56) References cited:
- WO-A1-2007/095958
- US-A1- 2006 240 539
- ROBITAILLE G ET AL: "Fat-free yogurt made using a galactose-positive exopolysaccharide-producing recombinant strain of Streptococcus thermophilus", JOURNAL OF DAIRY SCIENCE, AMERICAN DAIRY SCIENCE ASSOCIATION, US, vol. 92, no. 2, 1 February 2009 (2009-02-01), pages 477 - 482, XP026955226, ISSN: 0022-0302, [retrieved on 20090201]
- ROBITAILLE G ET AL: "Galactose Metabolism and Capsule Formation in a Recombinant Strain of Streptococcus thermophilus with a Galactose-Fermenting Phenotype", JOURNAL OF DAIRY SCIENCE, AMERICAN DAIRY SCIENCE ASSOCIATION, US, vol. 90, no. 9, 1 September 2007 (2007-09-01), pages 4051 - 4057, XP026956143, ISSN: 0022-0302, [retrieved on 20070901]
- VAILLANCOURT KATY ET AL: "Characterization of a galactokinase-positive recombinant strain of Streptococcus thermophilus.", APPLIED AND ENVIRONMENTAL MICROBIOLOGY AUG 2004 LNKD- PUBMED:15294791, vol. 70, no. 8, August 2004 (2004-08-01), pages 4596 - 4603, XP002604685, ISSN: 0099-2240
- DE VIN FILIP ET AL: "Molecular and biochemical analysis of the galactose phenotype of dairy Streptococcus thermophilus strains reveals four different fermentation profiles", APPLIED AND ENVIRONMENTAL MICROBIOLOGY, vol. 71, no. 7, July 2005 (2005-07-01), pages 3659 - 3667, XP002604686, ISSN: 0099-2240
- MOZZI F ET AL: "Effect of galactose and glucose on the exopolysaccharide production and the activities of biosynthetic enzymes in Lactobacillus casei CRL 87", JOURNAL OF APPLIED MICROBIOLOGY, vol. 91, no. 1, July 2001 (2001-07-01), pages 160 - 167, XP002604687, ISSN: 1364-5072
- HASSAN A N ET AL: "Microstructure and rheology of yogurt made with cultures differing only in their ability to produce exopolysaccharides", JOURNAL OF DAIRY SCIENCE, AMERICAN DAIRY SCIENCE ASSOCIATION, US LNKD- DOI:10.3168/JDS.S0022-0302(03)73748-5, vol. 86, no. 5, 1 May 2003 (2003-05-01), pages 1632 - 1638, XP002377643, ISSN: 0022-0302
- BROADBENT J R ET AL: "Biochemistry, genetics, and applications of exopolysaccharide production in Streptococcus thermophilus: a review", JOURNAL OF DAIRY SCIENCE, AMERICAN DAIRY SCIENCE ASSOCIATION, US LNKD- DOI:10.3168/JDS.S0022-0302(03)73619-4, vol. 86, no. 2, 1 February 2003 (2003-02-01), pages 407 - 423, XP009018436, ISSN: 0022-0302

### SEQUENCE LISTING

<110> Chr. Hansen A/S
<120> Bacterium
<130> P3652PC00
<150> DK 2009 00984
   <151> 2009-09-01
<160> 8
<170> PatentIn version 3.3
<210> 1
   <211> 6
   <212> DNA
   <213> Streptococcus thermophilus
<400> 1
   tacgat 6
<210> 2
   <211> 6
   <212> DNA
   <213> Streptococcus thermophilus
<400> 2
   tatgat 6
<210> 3
   <211> 6
   <212> DNA
<213> Streptococcus thermophilus
<400> 3
   tattat 6
<210> 4
   <211> 6
   <212> DNA
   <213> Streptococcus thermophilus
<400> 4
   tactat 6
<210> 5
   <211> 70
   <212> DNA
   <213> Streptococcus thermophilus
<400> 5
   aaaatattga ttttccatgt gaaaggggtt atgatttcag tataaacaaa aagaataagt     60
   gagatacatc                                                            70
<210> 6
   <211> 70
   <212> DNA
   <213> Streptococcus thermophilus
<400> 6
   aaaatattga ttttccatgt gaaaggggtt acgatttcag tataaacaaa aagaataagt     60
   gagatacatc                                                            70
<210> 7
   <211> 70
   <212> DNA
   <213> Streptococcus thermophilus
<400> 7
   aaaatattga ttttccatgt gaaaggggtt acgatttcag tataaacaaa aagaataagt     60
   gagatacatc                                                            70
<210> 8
   <211> 70
   <212> DNA
   <213> Streptococcus thermophilus
<400> 8
   aaaatattga ttttccatgt gaaaggggtt acgatttcag tataaacaaa aagaataagt     60
   gagatacatc                                                            70

## Description

### FIELD OF INVENTION

The present invention relates to a bacterial cell with texturizing property, starter cultures comprising the cell, and dairy products fermented with the starter culture.

### BACKGROUND OF INVENTION

The food industry uses numerous bacteria, in particular lactic bacteria, in order to improve the taste and the texture of foods but also in order to extend the shelf life of these foods. In the case of the dairy industry, lactic bacteria are used intensively in order to bring about the acidification of milk (by fermentation) but also in order to texturize the product into which they are incorporated.

Among the lactic bacteria used in the food industry, there can be mentioned the genera *Streptococcus, Lactococcus, Lactobacillus, Leuconostoc, Pediococcus* and *Bifidobacterium.*
The lactic bacteria of the species *Streptococcus thermophilus* are used extensively alone or in combination with other bacteria for the production of food products, in particular fermented products. They are used in particular in the formulation of the ferments used for the production of fermented milks, for example yogurts. Certain of them play a dominant role in the development of the texture of the fermented product. This characteristic is closely linked to the production of polysaccharides. Among the strains of *Streptococcus thermophilus* it is possible to distinguish texturizing and non-texturizing strains.

In order to meet the requirements of the industry, it has become necessary to propose novel texturizing strains of lactic bacteria, in particular of *Streptococcus thermophilus.*

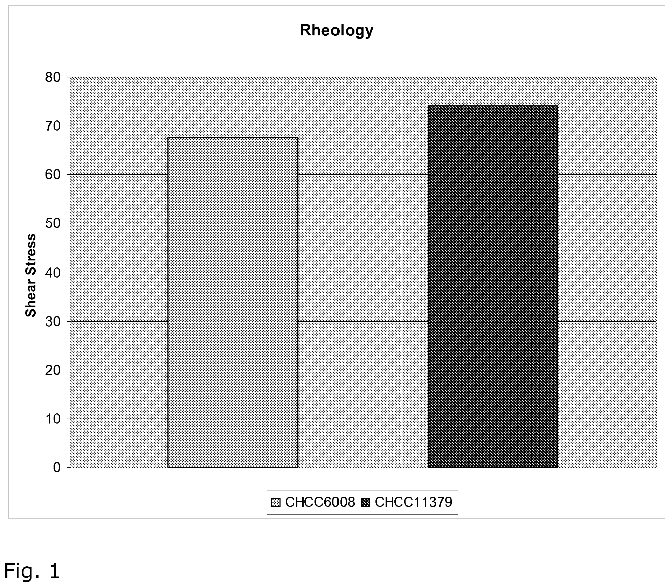

WO2007095958A1 discloses *Streptococcus thermophilus* strains with texturising properties. In figure 1 it can be seen that the most texturizing strain CHCC8833 (DSM17876) has a shear stress value of around 59 Pa.

Robitaille et al., J Dairy Sci. 92: 477-482 (2009) discloses a study which shows that it is possible to increase the production of EPS by ropy *Streptococcus thermophilus* strains through genetic engineering by increasing GalK activity, i.e. by transformation with a plasmid overexpressing GalK. It is concluded that when such a strain is used in combination with *Lactobacillus bulgaricus* for yogurt manufacture, the EPS overproduction of the recombinant strain is not significant. Robitaille et al., J Dairy Sci. 90(9):4051-4057 (2007) and Vaillancourt et al., App Envir Microbiol. 70(8):4596-4603 (2004) also disclose the production of Galactose-positive derivatives of *Streptococcus thermophilus* by transformation with a plasmid overexpressing GalK of *Strep. salivarius.* The modified strains did either not show a significant increase in EPS production in milk (Robitaille) or the EPS production was not analyzed (Vaillancourt). De Vin et al., Appl Environ Microbiol. 71(7):3659-3667 (2005) discloses a molecular and biochemical analysis of the galactose phenotype of various dairy *Streptococcus thermophilus* strains, *inter alia* of the nucleotide sequences as found in the *galR-galK* intergenic region which includes the -10 region.

Thus the problem that the invention proposes to resolve is to provide a strain of lactic bacterium having good properties for texturizing food products, especially products where the texturizing strain of the lactic bacterium (such as *Streptococcus thermophilus*) is used together with a strain of a *Lactobacillus* species.

### SUMMARY OF INVENTION

The present inventors have surprisingly found that lactic acid bacteria with mutations in the GalK (galactokinase) gene generates a higher viscosity in fermented milk than the wild type (mother) strain, especially when used for fermenting milk together with *Lactobacillus* bacteria.

In accordance with this surprising finding, the present invention relates to novel supertexturizing lactic acid bacteria stains, such as S. *thermophilus* strains, with mutations in the GalK gene, and a method for producing these strains, and to fermented milk products made using such strains.

### DETAILED DISCLOSURE

In a first aspect, the present invention relates to a method for manufacturing a lactic acid bacterium (such as a bacterium which generates higher viscosity in fermented milk compared to the mother stain, or such as a bacterium which generates a viscosity in fermented milk greater than about 70 Pa (e.g. greater than 71 or greater than 73 Pa, measured as shear stress after 12 hours growth at 37 degrees C), comprising:
a) Providing a lactic acid bacterial strain as the mother strain;
b) introducing a mutation in the GalK gene of the strain; and
c) screening for a mutant strain which generates a viscosity in fermented milk greater
than about 70 Pa measured as shear stress after 12 hours of growth at 37 degrees.

Thus, embodiments of the first aspect are:
- A method for manufacturing a lactic acid bacterium which generates higher viscosity in fermented milk compared to the mother stain (e.g. measured as shear stress, such as after 12 hours growth at 37 degrees C) comprising:
   a) Providing a lactic acid bacterial strain as the mother strain;
   b) introducing a mutation in the GalK gene of the strain; and
   c) screening for a mutant strain which generates a viscosity in fermented milk greater than about 70 Pa measured as shear stress after 12 hours of growth at 37 degrees.
- A method for manufacturing a lactic acid bacterium which generates a viscosity in fermented milk greater than about 70 Pa (e.g. measured as shear stress, such as after 12 hours growth at 37 degrees C) comprising:
   a) Providing a lactic acid bacterial strain as the mother strain;
   b) introducing a mutation in the GalK gene of the strain; and
   c) screening for a mutant strain which generates a viscosity in fermented milk greater than about 70 Pa measured as shear stress after 12 hours of growth at 37 degrees.

In presently preferred embodiments, the viscosity is measured as shear stress after 12 hours growth at 37 degrees C, and/or as measured in the assay described below. Thus, a presently preferred embodiment of the first aspect of the invention is a method for manufacturing a lactic acid bacterium comprising:
a) providing a lactic acid bacterial strain as the mother strain;
b) introducing a mutation in the GalK gene of the strain; and
c) screening for a mutant strain which generates higher viscosity in fermented milk compared to the mother strain measured as shear stress after 12 hours of growth at 37 degrees C.

The method of the invention may further comprise one or more further step(s) selected from the group consisting of:
c1) screening for a mutant strain which generates texture, such as a strain which generates more texture than the mother stain, or increases the viscosity of a milk substrate; and
c2) screening for a mutant strain having Gal+ phenotype, such as improved galactose degrading/fermenting activity compared to the mother stain.

The mutation may be introduced in the promoter region of the gene, such as in the -10 region (the Pribnow box) of the gene.

In an interesting embodiment of the method of the invention, the mutation results in the replacement of one or both of C and G in the wildtype -10 region which has the nucleotide sequence TACGAT with a nucleotide independently selected from the group consisting of A and T.

The mutation may result in the replacement of C the wildtype -10 region (TACGAT) with a nucleotide independently selected from the group consisting of A and T.

In an embodiment, the mutation results in the replacement of C the wildtype -10 region which has the nucleotide sequence TACGAT with T, and/or the mutation results in a -10 region which has the nucleotide sequence TATGAT, TATTAT or TACTAT.

The mutation may be introduced by use of genetic engineering techniques, and/or by use of mutagenesis, e.g. by radiation or treatment with a chemical, optionally followed by an analysis of the GalK gene in order to verify mutation in the GalK gene.

The bacterium (the mother strain) may be selected from a genus belonging to the group consisting of *Streptococcus, Lactococcus, Lactobacillus, Leuconostoc, Pediococcus,* and *Bifidobacterium.* An interesting bacterium belongs to the species *Streptococcus thermophilus.*

Further disclosed is a lactic acid bacterium which is obtainable by the method of the invention.

Also, the invention relates to a lactic acid bacterium which generates a viscosity in fermented milk greater than about 70 Pa (such as greater than 71 Pa or 73 Pa), measured as shear stress after 12 hours of growth at 37 degrees C. In a further aspect, the present invention relates to a lactic acid bacterium which carries a mutation in the -10 region of the GalK gene (compared to the wildtype -10 region which has the nucleotide sequence TACGAT), wherein the mutation results in a -10 region which has the nucleotide sequence TATTAT (SEQ ID NO:3) or TACTAT (SEQ ID NO:4) and wherein the lactic acid bacterium is obtainable by the method of claim 1, wherein the lactic acid bacterium generates higher viscosity in fermented milk compared to the mother strain measured as shear stress after 12 hours of growth at 37 degrees C, the mother strain being CHCC6008, deposited at the DSMZ - Deutsche Sammlung von Mikroorganismen und Zellkulturen GmbH under the accession number DSM 18111. Further disclosed is a lactic acid bacterium which carries a mutation in the -10 region of the GalK gene, which mutation results in the replacement of the C in the wildtype -10 region, which has the nucleotide sequence TACGAT, with a nucleotide independently selected from the group consisting of A and T. The mutation may result in the replacement of the C in the wildtype -10 region, which has the nucleotide sequence TACGAT, with T.

In an interesting embodiment, a lactic acid bacterium of the invention comprises SEQ ID NO:5, or the promoter region thereof, including the -35 region and the -10 region. The lactic acid bacterium preferably belongs to the species *Streptococcus thermophilus.*

In a further embodiment, the invention relates to a bacterial strain belonging to the species *Streptococcus thermophilus,* selected from the group consisting of: CHCC11379 (DSM 22884), CHCC11342 (DSM 22932) and CHCC11976 (DSM 22934). Disclosed are also all novel strains mentioned herein, as well as their mutants.

In a further aspect, the invention relates to a composition comprising lactic acid bacteria of the invention, such as bacteria belonging to the strain CHCC11379 (DSM 22884). It is preferred that such composition comprises at least 10exp10 CFU (cell forming units) of said bacteria.

The composition may comprise, either as a mixture or as a kit-of-parts,
- a strain belonging to a *Lactobacillus* species, such as a *L. bulgaricus* or a *L. fermentum* strain; and
- a strain of a lactic acid bacterium of the invention, such as a strain belonging to the species *Streptococcus thermophilus.*

It is presently preferred that the strain belonging to a *Lactobacillus* species is a strain belonging to a polysaccharide (such as a heteropolysaccharide, homopolysaccharide) and/or fructosyl transferase enzyme producing *Lactobacillus* species.

The composition may comprise at least 10exp10 CFU (cell forming units) of a strain belonging to a *Lactobacillus* species; and at least 10exp10 CFU of a strain belonging to the species *Streptococcus thermophilus.*

The composition is preferably usable as a starter culture, and is in frozen, freeze-dried or liquid form.

In an other aspect, the invention relates to a method for producing a fermented milk product/dairy product, comprising fermenting a milk substrate (such as cow's milk) with a lactic acid bacterium, a strain, or a composition of the invention.

The method may further comprise fermenting the milk substrate with a strain belonging to a *Lactobacillus species,* such as a strain of *L. bulgaricus* or *L. fermentum,* e.g. a strain selected from the group consisting of LB18, CHCC10019 (DSM19252), CHCC2008 (DSM22584), or CHCC3984 (DSM19251). Disclosed are also mutants and variants of any of these strains.

The milk substrate may fermented with a strain or bacterium of any preceding claim, such as a strain belonging to the species *Streptococcus thermophilus* before, during, or after the fermentation with a strain belonging to a *Lactobacillus* species.

The method may comprise adding an enzyme to the milk substrate before, during and/or after the fermenting, such as an enzyme selected from the group consisting of: an enzyme able to crosslink proteins, transglutaminase, an aspartic protease, chymosin, and rennet.

In an other aspect, the present invention relates to a dairy product, such as a fermented milk product (e.g. yoghurt or buttermilk) or a cheese (e.g. fresh cheese or pasta filata), obtainable by the method of the invention. The fermented milk product may e.g. be a stirred-type product, or a set-type product.

The dairy product may optionally comprise an ingredient selected from the group consisting of: a fruit concentrate, a syrup, a probiotic bacterial culture, a coloring agent, a thickening agent, a flavoring agent, and a preserving agent; and/or which optionally is in the form of a stirred type product, a set type product, or a drinkable product.

Also the invention relates to a dairy product, which is made by fermenting a milk substrate (such as cow's milk) with a lactic acid bacterium of the invention (e.g. a strain belonging to the species *Streptococcus thermophilus,* such as DSM 22884) and a lactic acid bacterium a species selected from *Lactobacillus bulgaricus* and *Lactobacillus fermentum* (such as CHCC10019 (DSM19252), CHCC3984 (DSM19251) and CHCC2008 (DSM22584)).

In interesting a dairy product of the invention has a viscosity of more than 100 Pa (such as more than 102 or more than 104 Pa), measured as shear stress, e.g. after 12 hours growth at 37 degrees C. In a presently preferred embodiment, the viscosity of more than 100 Pa is obtained by growth of the bacterial cells alone, but higher viscosity values can be obtained by addition of chemical compounds, such as gelatine, a carrageenan, etc.

### DEFINITIONS

As used herein, the term "lactic acid bacterium" designates a gram-positive, microaerophilic or anaerobic bacterium, which ferments sugars with the production of acids including lactic acid as the predominantly produced acid, acetic acid and propionic acid. The industrially most useful lactic acid bacteria are found within the order "Lactobacillales" which includes *Lactococcus* spp., *Streptococcus* spp., *Lactobacillus* spp., *Leuconostoc* spp., *Pseudoleuconostoc* spp., *Pediococcus* spp., *Brevibacterium* spp., *Enterococcus* spp. and *Propionibacterium* spp. Additionally, lactic acid producing bacteria belonging to the group of the strict anaerobic bacteria, bifidobacteria, i.e. *Bifidobacterium* spp., are generally included in the group of lactic acid bacteria. These are frequently used as food cultures alone or in combination with other lactic acid bacteria. Lactic acid bacteria, including bacteria of the species Lactobacillus sp. and Streptococcus thermophilus, are normally supplied to the dairy industry either as frozen or freeze-dried cultures for bulk starter propagation or as so-called "Direct Vat Set" (DVS) cultures, intended for direct inoculation into a fermentation vessel or vat for the production of a dairy product, such as a fermented milk product. Such cultures are in general referred to as "starter cultures" or "starters".

The term "milk" is to be understood as the lacteal secretion obtained by milking any mammal, such as cows, sheep, goats, buffaloes or camels. In a preferred embodiment, the milk is cow's milk. The term milk also includes protein/fat solutions made of plant materials, e.g. soy milk.

The term "milk substrate" may be any raw and/or processed milk material that can be subjected to fermentation according to the method of the invention. Thus, useful milk substrates include, but are not limited to, solutions/suspensions of any milk or milk like products comprising protein, such as whole or low fat milk, skim milk, buttermilk, reconstituted milk powder, condensed milk, dried milk, whey, whey permeate, lactose, mother liquid from crystallization of lactose, whey protein concentrate, or cream. Obviously, the milk substrate may originate from any mammal, e.g. being substantially pure mammalian milk, or reconstituted milk powder.

Prior to fermentation, the milk substrate may be homogenized and pasteurized according to methods known in the art.

"Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices.

"Pasteurizing" as used herein means treatment of the milk substrate to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

"Fermentation" in the methods of the present invention means the conversion of carbohydrates into alcohols or acids through the action of a microorganism. Preferably, fermentation in the methods of the invention comprises conversion of lactose to lactic acid.

Fermentation processes to be used in production of fermented milk products are well known and the person of skill in the art will know how to select suitable process conditions, such as temperature, oxygen, amount and characteristics of microorganism(s) and process time. Obviously, fermentation conditions are selected so as to support the achievement of the present invention, i.e. to obtain a dairy product in solid or liquid form (fermented milk product).

The term "stirred type product" specifically refers to a fermented milk product which sustains a mechanical treatment after fermentation, resulting in a destructuration and liquefaction of the coagulum formed under the fermentation stage. The mechanical treatment is typically but not exclusively obtained by stirring, pumping, filtrating or homogenizing the gel, or by mixing it with other ingredients. Stirred type products typically but not exclusively have a milk solid non-fat content of 9 to 15%.

The term "set-type product" includes a product based on milk which has been inoculated with a starter culture, e.g. a starter culture, and packaged next to the inoculating step and then fermented in the package.

In the present context, the term "mutant" should be understood as a strain derived from a strain of the invention by means of e.g. genetic engineering, radiation and/or chemical treatment. It is preferred that the mutant is a functionally equivalent mutant, e.g. a mutant that has substantially the same, or improved, properties (e.g. regarding texture, shear stress, viscosity, gel stiffness, mouth coating, flavor, post acidification, acidification speed, and/or phage robustness) as the mother strain. According to the invention, the mutant generates a viscosity in fermented milk greater than about 70 Pa measured as shear stress after 12 hours of growth at 37 degrees. Such a mutant is a part of the present invention. Especially, the term "mutant" refers to a strain obtained by subjecting a strain of the invention to any conventionally used mutagenization treatment including treatment with a chemical mutagen such as ethane methane sulphonate (EMS) or N-methyl-N'-nitro-N-nitroguanidine (NTG), UV light or to a spontaneously occurring mutant. A mutant may have been subjected to several mutagenization treatments (a single treatment should be understood one mutagenization step followed by a screening/selection step), but it is presently preferred that no more than 20, or no more than 10, or no more than 5, treatments (or screening/selection steps) are carried out. In a disclosed mutant, less that 5%, or less than 1% or even less than 0.1% of the nucleotides in the bacterial genome have been shifted with another nucleotide, or deleted, compared to the mother strain. In the present context, the term "variant" should be understood as a strain which is functionally equivalent to a strain of the invention, e.g. having substantially the same, or improved, properties e.g. regarding texture, shear stress, viscosity, gel stiffness, mouth coating, flavor, post acidification, acidification speed, and/or phage robustness). Such variants, which may be identified using appropriate screening techniques, are disclosed herein.

In the present context, "texture" is measured as shear stress after 12 hours growth at 37 degrees C. An assay to be used for analysis of texture:
The day after incubation, the fermented milk was brought to 13°C and stirred gently by means of a stick fitted with a bored disc until homogeneity of the sample. The rheological properties of the sample were assessed on a rheometer (StressTech, Reologica Instruments, Sweden) equipped with a C25 coaxial measuring system. The viscometry test was made with shear rates varying from 0.27 to 300 1/s in 21 steps. Shear rates were increased and then decreased and the upward and downward curves of shear stress and apparent viscosity were recorded. Delay and integration times were 5 s and 10 s, respectively.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

### LEGENDS

Figure 1 depicts the shear stress of CHCC11379 measured with the StressTech rheometer. Result showing improved texture (shear stress) in milk coagulated with CHCC11379 after over/night growth at 43 degrees C. Texture was quantified by assessing shear stress measured in Pascals (Pa) by use of a StressTech rheometer as described under "Analysis of texture in fermented milk"

### EXAMPLES

### Example 1 Preparation of a galactose positive mutant of a Streptococcus strain

### Isolation of galactose fermenting strain

Mutants were isolated as galactose fermenting mutant of *S. thermophilus* strain CHCC6008 (=ST6008, DSM18111). The CHCC6008 cells were neither mutagenized with any mutagenic compound nor by UV light prior to the mutant isolation step. The isolated strains resemble therefore spontaneous galactose positive mutants of CHCC6008.

Prior to the mutant isolation CHCC6008 was streaked on M17 agar plates with 2% galactose (M17-gal plates). CHCC6008 did not grow on galactose as sole carbohydrate source.

Over night culture of CHCC6008 were then plated on M17-gal plates and several colonies could be isolated after two days of growth at 37°C. Several mutants were purified on M17-gal plates and retested in M17 broth containing 2% galactose as sole carbohydrate.

Whereas CHCC6008 did not lower the pH in M17-gal broth significantly, CHCC11379, one of the purified mutants, reached a pH of 5.3 after 10 hours at 37 °C, and was therefore considered a galactose-fermenting mutant from CHCC6008.

Strains CHCC11342 and CHCC11976 were isolated the same way.

### Isolation of mutants by genetic engineering

Galactose positive mutants can also be generated by site directed mutagenesis. Oligonucleotides carrying the mutated nucleotide within the galK -10 promoter box is used to amplify a specific DNA fragment by PCR. The PCR fragment carrying the desired mutation is cloned into a vector plasmid and transformed into the S. *thermophilus* target strain, and the mutation is integrated into the chromosome and exchanging the wild type galK promoter region by recombination. Isolation of strains is done as above.

### Analysis of texture in fermented milk

The day after incubation, the fermented milk was brought to 13°C and stirred gently by means of a stick fitted with a bored disc until homogeneity of the sample. The rheological properties of the sample were assessed on a rheometer (StressTech, Reologica Instruments, Sweden) equipped with a C25 coaxial measuring system.

The viscometry test was made with shear rates varying from 0.27 to 300 1/s in 21 steps. Shear rates were increased and then decreased and the upward and downward curves of shear stress and apparent viscosity were recorded.

Delay and integration times were 5 s and 10 s, respectively. For further analysis, shear stress at 300 s-1 was chosen. The rheometer results showed that CHCC11379 had a shear stress which was improved by 10% compared to CHCC6008 (shear stress value 74.0 Pa (Pascals) for CHCC11379 compared with 67.5 Pa for mother strain CHCC6008, see Fig. 1).

### Analysis of texture in milk

In another experiment CHCC11379 was used as a part of a yoghurt culture where strains from S. *thermophilus* are co-cultured in skimmed milk at 43 °C together with a strain from the species *Lactobacillus delbrueckii* ssp. *bulgaricus.* When the only difference in the production of yoghurt was the use of CHCC11379 instead of wild type CHCC6008 the shear stress was also increased by 10% (105.0 Pa for the yoghurt culture containing CHCC11379 compared with 94.7 Pa for the yoghurt culture containing CHCC6008).

### Sequenceing of the galK promoter region from CHCC11379

To reveal the type of mutation for the gal positive mutant CHCC11379 the beginning of the *galK* gene (coding for the galactokinase from S. *thermophilus*) was sequenced.

For CHCC11379 a mutation in the region of the *galK* promoter was identified (see below). The respective mutation will most likely lead to a stronger promoter activity compared to the mother strain 6008, explaining the observed gal-positive phenotype. This is based on the fact that the consensus sequence for the -10-promoter box is "TATAAT", and that a mutation at nucleotide 3 of the -10 box (region) for CHCC6008 ("TACGAT") leads to a -10 box with a higher similarity to the consensus sequence in CHCC11379 ("TATGAT").

Promoter region of the *galK* gene from CHCC11379. The point mutation within the CHCC11379 *galk* promoter is indicated with grey color code. The published *galK* sequence from *S. thermophilus* ST111 (Genbank accession no. AY704368) is indicated for comparison. -35: -35-promoter region; -10: -10-promoter region; RBS: ribosome binding site.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

### DEPOSITS and EXPERT SOLUTION

The strain *Streptococcus thermophilus* CHCC11379 has been deposited at DSMZ (Deutsche Sammlung von Mikroorganismen und Zellkulturen GmbH, Inhoffenstr. 7B, D-38124 Braunschweig, Germany) under the accession number DSM22884, the 26 August 2009.

A sample of the Streptococcus thermophilus (ST) strain ST6008 (also called CHCC6008) has been deposited at DSMZ under the accession number DSM 18111 with a deposit date of 29th March 2006. CHCC11342 (DSM 22932) and CHCC11976 (DSM 22934) were deposited 8 Sept 2009 at DSMZ.

Further deposits at DSMZ:
CHCC10019 (DSM19252), CHCC3984 (DSM19251): Date of deposit 3 april 2007,
CHCC2008 (DSM22584): date of deposit 19 may 2009,

The deposits have been made under the conditions of the Budapest Treaty on the International Recognition of the Deposit of Microorganisms for the Purposes of Patent Procedure.

The Applicant requests that a sample of the deposited microorganisms should be made available only to an expert approved by the Applicant.

### REFERENCES

Appl. Environ, Microbiol. 71, 7, p. 3659-67 (2005)
International Journal of Food Microbiology 113 (2007) 195-200
APPLIED AND ENVIRONMENTAL MICROBIOLOGY, Feb. 2002, p. 784-790
J Dairy Sci 92: 477-482 (2009)
WO2008/040734A1, WO2007/025097A2, US7241610B2, WO2007/144770A2,
WO2004/085607A, WO2008/148561A

## Claims

1. A method for manufacturing a lactic acid bacterium comprising:
a) providing a lactic acid bacterial strain as the mother strain;
b) introducing a mutation in the GalK gene of the strain; and
c) screening for a mutant strain which generates higher viscosity in fermented milk compared to the mother strain measured as shear stress after 12 hours of growth at 37 degrees C.

2. The method of claim 1, wherein the mutation results in the replacement of one or both of C and G in the wildtype -10 region which has the nucleotide sequence TACGAT (SEQ ID NO:1) with a nucleotide independently selected from the group consisting of A and T.

3. The method of claim 2, wherein the mutation results in a -10 region which has the nucleotide sequence TATGAT (SEQ ID NO:2), TATTAT (SEQ ID NO:3) or TACTAT (SEQ ID NO:4).

4. The method of any of the preceding claims, wherein the bacterium belongs to the species *Streptococcus thermophilus.*

5. A lactic acid bacterium which carries a mutation in the -10 region of the GalK gene, wherein the mutation results in a -10 region which has the nucleotide sequence TATTAT (SEQ ID NO:3) or TACTAT (SEQ ID NO:4) and wherein the lactic acid bacterium is obtainable by the method of claim 1, wherein the lactic acid bacterium generates higher viscosity in fermented milk compared to the mother strain measured as shear stress after 12 hours of growth at 37 degrees C, the mother strain being CHCC6008, deposited at the DSMZ - Deutsche Sammlung von Mikroorganismen und Zellkulturen GmbH under the accession number DSM 18111.

6. A bacterial strain belonging to the species *Streptococcus thermophilus,* selected from the group consisting of: CHCC11379 deposited at the DSMZ - Deutsche Sammlung von Mikroorganismen und Zellkulturen GmbH under the accession number DSM 22884, CHCC11342 deposited at the DSMZ - Deutsche Sammlung von Mikroorganismen und Zellkulturen GmbH under the accession number DSM 22932, and CHCC11976 deposited at the DSMZ - Deutsche Sammlung von Mikroorganismen und Zellkulturen GmbH under the accession number DSM 22934.

7. A composition comprising a lactic acid bacterium of claim 5 or a bacterial strain of claim 6.

8. A composition comprising, either as a mixture or as a kit-of-parts,
- a strain of a lactic acid bacterium of claim 5 or a bacterial strain of claim 6; and
- a strain belonging to a *Lactobacillus* species.

9. A method for producing a fermented milk product/dairy product, comprising fermenting a milk substrate with a lactic acid bacterium of claim 5, a bacterial strain of claim 6, or a composition of any of claims 7-8.

10. The method of claim 9, wherein said *Lactobacillus species* is a strain of *L. bulgaricus* or *L. fermentum,* preferably a strain selected from the group consisting of LB18, CHCC10019 deposited at the DSMZ - Deutsche Sammlung von Mikroorganismen und Zellkulturen GmbH under the accession number DSM19252, CHCC2008 deposited at the DSMZ - Deutsche Sammlung von Mikroorganismen und Zellkulturen GmbH under the accession number DSM22584, or CHCC3984 deposited at the DSMZ - Deutsche Sammlung von Mikroorganismen und Zellkulturen GmbH under the accession number DSM19251.

11. A dairy product or fermented milk product comprising the lactic acid bacterium of claim 5 or the bacterial strain of claim 6.

## Patentansprüche

1. Verfahren zur Herstellung eines Milchsäurebakteriums mit:
a) Bereitstellung eines Milchsäurebakterienstamms als Mutterstamm;
b) Einführung einer Mutation in das GalK-Gen des Stammes; und
c) Suche nach einem Mutantenstamm, der in fermentierter Milch eine größere Viskosität, im Vergleich zum Mutterstamm, erzeugt, gemessen als Scherspannung nach 12 Stunden Wachstum bei 37 Grad C.

2. Verfahren nach Anspruch 1, wobei die Mutation zum Austausch von einem oder beiden von C und G im Wildtyp - 10-Bereich führt, der die Nukleotidsequenz TACGAT (SEQ ID NO: 1) hat, mit einem Nukleotid, das unabhängig aus der Gruppe bestehend aus A und T ausgewählt wurde.

3. Verfahren nach Anspruch 2, wobei die Mutation zu einem -10-Bereich führt, der die Nukleotidsequenz TATGAT (SEQ ID NO: 2), TATTAT (SEQ ID NO: 3) oder TACTAT (SEQ ID NO: 4) hat.

4. Verfahren nach einem beliebigen der vorhergehenden Ansprüche, wobei das Bakterium der Art *Streptococcus thermophilus* angehört.

5. Milchsäurebakterium, das eine Mutation in dem -10-Bereich des GalK-Gens trägt, wobei die Mutation zu einem -10 Bereich führt, der die Nukleotidsequenz TATTAT (SEQ ID NO:3) oder TACTAT (SEQ ID NO:4) hat und wobei das Milchsäurebakterium durch das Verfahren nach Anspruch 1 erhältlich ist, wobei das Milchsäurebakterium eine höhere Viskosität in Milch im Vergleich zum Mutterstamm erzeugt, gemessen als Scherspannung nach 12 Stunden Wachstum bei 37 Grad C, wobei der Mutterstamm CHCC6008, hinterlegt bei der DSMZ - Deutsche Sammlung von Mikroorganismen und Zellkulturen GmbH unter der Zugangszahl DSM 18111, ist.

6. Bakterienstamm der Art *Streptococcus thermophilus,* ausgewählt aus der Gruppe bestehend aus: CHCC11379, hinterlegt bei der DSMZ - Deutsche Sammlung von Mikroorganismen und Zellkulturen GmbH unter der Zugangszahl DSM 22884; CHCC11342, hinterlegt bei der DSMZ - Deutsche Sammlung von Mikroorganismen und Zellkulturen GmbH unter der Zugangszahl DSM 22932, und CHCC11976, hinterlegt bei der DSMZ - Deutsche Sammlung von Mikroorganismen und Zellkulturen GmbH unter der Zugangszahl DSM 22934.

7. Zusammensetzung mit einem Milchsäurebakterium nach Anspruch 5 oder einem Bakterienstamm nach Anspruch 6.

8. Zusammensetzung mit, entweder als Mischung oder als Bausatz,
- einem Stamm eines Milchsäurebakteriums nach Anspruch 5 oder einem Bakterienstamm nach Anspruch 6; und
- einem Stamm einer Lactobacillusart.

9. Verfahren zur Herstellung eines fermentierten Milchprodukts/Molkereiprodukts, mit Fermentierung eines Milchsubstrats mit einem Milchsäurebakteriums nach Anspruch 5, einem Bakterienstamm nach Anspruch 6 oder einer Zusammensetzung nach einem beliebigen der Ansprüche 7-8.

10. Verfahren nach Anspruch 9, wobei besagte Lactobacillusart ein Stamm von *L. bulgaricus* oder *L. fermentum* ist, vorzugsweise ein Stamm ausgewählt aus der Gruppe bestehend aus: LB18, CHCC10019, hinterlegt bei der DSMZ - Deutsche Sammlung von Mikroorganismen und Zellkulturen GmbH unter der Zugangszahl DSM19252; CHCC2008, hinterlegt bei der DSMZ - Deutsche Sammlung von Mikroorganismen und Zellkulturen GmbH unter der Zugangszahl DSM22584, oder CHCC3984, hinterlegt bei der DSMZ - Deutsche Sammlung von Mikroorganismen und Zellkulturen GmbH unter der Zugangszahl DSM19251.

11. Molkereiprodukt oder fermentiertes Milchprodukt mit dem Milchsäurebakterium nach Anspruch 5 oder dem Bakterienstamm nach Anspruch 6.

## Revendications

1. Procédé de fabrication d'une bactérie lactique comprenant :
a) la fourniture d'une souche de bactérie lactique en tant que souche mère ;
b) l'introduction d'une mutation dans le gène GalK de la souche ; et
c) le filtrage pour une souche mutante qui génère une viscosité plus élevée dans le lait fermenté par rapport à la souche mère mesurée comme contrainte de cisaillement après 12 heures de croissance à 37 degrés Celsius.

2. Procédé selon la revendication 1, dans lequel la mutation aboutit au remplacement de l'un entre C et G ou les deux dans la région de type sauvage -10 qui a la séquence nucléotidique TACGAT (SEQ ID NO: 1) avec un nucléotide indépendamment sélectionné du groupe composé de A et T.

3. Procédé selon la revendication 2, dans lequel la mutation aboutit à une région -10 qui a la séquence nucléotidique TATGAT (SEQ ID NO:2), TATTAT (SEQ ID NO:3) ou TACTAT (SEQ ID NO:4).

4. Procédé selon l'une quelconque des revendications précédentes, dans lequel la bactérie appartient à l'espèce *Streptococcus thermophilus.*

5. Bactérie d'acide lactique qui porte une mutation dans la région -10 du gène GalK, dans lequel la mutation aboutit à une région -10 qui a la séquence nucléotidique TATTAT (SEQ ID NO:3) ou TACTAT (SEQ ID NO:4) et dans lequel la bactérie d'acide lactique peut être obtenue par le procédé de la revendication 1, dans lequel la bactérie d'acide lactique génère une viscosité plus élevée dans du lait fermenté par rapport à la souche mère mesurée comme contrainte de cisaillement après 12 heures de croissance à 37 degrés Celsius, la souche mère étant CHCC6008, déposée auprès de la DSMZ - Deutsche Sammlung von Mikroorganismen und Zellkulturen GmbH sous le numéro d'entrée DSM 18111.

6. Souche de bactérie appartenant à l'espèce *Streptococcus thermophilus,* sélectionnée parmi le groupe consistant en : CHCC11379 déposée auprès de la DSMZ - Deutsche Sammlung von Mikroorganismen und Zellkulturen GmbH sous le numéro d'entrée DSM 22884, CHCC11342 déposée auprès de la DSMZ - Deutsche Sammlung von Mikroorganismen und Zellkulturen GmbH sous le numéro d'entrée DSM 22932, et CHCC11976 déposée auprès de la DSMZ - Deutsche Sammlung von Mikroorganismen und Zellkulturen GmbH sous le numéro d'entrée DSM 22934.

7. Composition comprenant une bactérie d'acide lactique selon la revendication 5 ou une souche de bactérie selon la revendication 6.

8. Composition comprenant, soit comme un mélange ou comme un ensemble de composants,
- une souche d'une bactérie d'acide lactique selon la revendication 5 ou une souche bactérienne selon la revendication 6 ; et
- une souche appartenant à une espèce *Lactobacillus.*

9. Procédé pour produire un produit de lait fermenté/produit laitier, comprenant la fermentation d'un substrat de lait avec une bactérie d'acide lactique selon la revendication 5, une souche bactérienne selon la revendication 6, ou une composition selon l'une quelconque des revendications 7 à 8.

10. Procédé selon la revendication 9, dans lequel ladite *espèce Lactobacillus* est une souche de *L. bulgaricus* ou *L. fermentum,* de préférence une souche sélectionnée du groupe consistant en LB18, CHCC10019 déposée auprès de la DSMZ - Deutsche Sammlung von Mikroorganismen und Zellkulturen GmbH sous le numéro d'entrée DSM 19252, CHCC2008 déposée auprès de la DSMZ - Deutsche Sammlung von Mikroorganismen und Zellkulturen GmbH sous le numéro d'entrée DSM 22584, ou CHCC3984 déposée auprès de la DSMZ - Deutsche Sammlung von Mikroorganismen und Zellkulturen GmbH sous le numéro d'entrée DSM 19251.

11. Produit laitier ou produit de lait fermenté comprenant la bactérie d'acide lactique selon la revendication 5 ou la souche bactérienne selon la revendication 6.
